Europäisches Patentamt

**European Patent Office** (11) Publication number: **0 225 871**
**B1**
Office européen des brevets

(12) **EUROPEAN PATENT SPECIFICATION**

(45) Date of publication of patent specification: **14.03.90** (51) Int. Cl.⁵: **A 61 N 1/30**

(21) Application number: **85903469.6**

(22) Date of filing: **10.06.85**

(86) International application number:
**PCT/US85/01074**

(87) International publication number:
**WO 86/07268 18.12.86 Gazette 86/27**

(54) **SYSTEM AND METHOD FOR CONTROLLING RATE OF ELECTROKINETIC DELIVERY OF A DRUG.**

(43) Date of publication of application:
**24.06.87 Bulletin 87/26**

(45) Publication of the grant of the patent:
**14.03.90 Bulletin 90/11**

(84) Designated Contracting States:
**AT BE CH DE FR GB IT LI LU NL SE**

(56) References cited:
**DE-A-3 300 947**
**FR-A-2 263 792**
**US-A-4 166 457**
**US-A-4 406 658**
**US-A-4 474 570**

(73) Proprietor: **Drug Delivery Systems Inc.**
**292 Madison Avenue**
**New York New York 10017 (US)**

(72) Inventor: **SIBALIS, Dan**
**268 Hallock Road**
**Stony Brook, NY 11790 (US)**

(74) Representative: **Patentanwälte Ruff und Beier**
**Neckarstrasse 50**
**D-7000 Stuttgart 1 (DE)**

## Description

The invention refers to a system for the delivery of a medicament or a drug through the skin or mucous membrane of a human or animal by electrokinetic action, comprising in combination drug reservoir means containing a drug, said drug reservoir means adapted to be in contact with said skin, said drug reservoir means retaining said drug and being capable of passing an electrical current through said drug, wherein said drug is passed through the skin by electrokinetic action at the first skin contact area, a pair of spaced first and second electrodes and circuit means for selecting the polarity of a power source with respect to the electrodes. Such system is already known (US-A-4 406 658).

Background of the Invention

It is known that the skin is permeable to certain compounds, such as nitroglycerine and scopolamine or the skin could be made more permeable by other means, such as by the use of solvents, or other types of enhancers, such as physical means or chemical enhancers. Thus, presently the rate of drug delivery is controlled by a semi-permeable membrane, for example, in an osmotic profusion type drug applicator. Such drug applicators are currently marketed and in use for the transdermal delivery of nitroglycerine and scopolamine. These systems, however, are limited just to drugs which are soluble in both water and oil, are of low molecular weight, and are potent in small concentrations. Consequently, there are very few drugs which can be utilized with this modality of delivery. In addition, it is very difficult to consistently produce a membrane or a matrix which ensures a predictable rate of drug delivery.

The delivery of a drug from a drug reservoir can be accomplished by electrokinetic effect, that is, either by iontophoresis, electrophoresis or by electro-osmosis. Further, the rate of delivery is affected by the amount of electric current applied between the skin and the drug reservoir. Further, the electric current affects both the skin of the patient and the drug reservoir independently so as to create conditions that increase or decrease the rate of delivery of the drug through the skin of the patient. For example, current will affect the pH of the skin at the interface between the membrane of the reservoir, or patch area. Also, a polarization of the skin will change skin permeability. The charge of the drug particles, or ionic charge, will cause repulsed direction of the ionized drug through the skin. Also, an electro-osmotic effect is created at the skin surface, or mucous membrane by electric current. As for the drug reservoir of the patch, electrical current passed through the drug, which is contained in the drug reservoir, can control or improve mobility of the drug thus allowing less current to be needed for drug movement into the skin or mucous membrane. Also, the natural diffusion of the drug compound acts within the patch and is controlled in either additive or subtractive fashion by sending an electric current through the drug independently of the current through the skin; the rate of delivery and the degree of concentration of the drug at the skin can be affected and independently controlled. Also, the pH of the drug within the patch near the skin can thus be changed by electric current. Also, electric current in the patch can cause electrolysis, thus activating in sequence pre-existing buffering agents in the drug reservoir. In summary, control of the rate of delivery of a drug from a drug reservoir through the skin can be controlled by independent but simultaneous currents through the patch and through the skin. It is hereby noted that descriptions relating to mass transfer activity by application of electric current is contained in the applicant's US-A-557 723, 4 622 031, 4 708 716, 4 640 689.

It is therefore an object of the present invention to control the rate of delivery of a drug from a drug reservoir by placing a plurality of electrodes within the reservoir in electrical circuit means with first and second power sources and with the skin or mucous-membrane, the patch of the first power source being optionally directed to one of the two electrodes so as to cause the second power source to be added or subtracted from the first power source for current directed through the skin.

Summary of the Invention

In order to achieve the objectives listed above and other objects that will become evident in the detailed description of the invention which follows, a system is provided for selectively controlling the rate of delivery of a medicament or drug through the skin or mucous membrane of a human or animal patient by electrokinetic action. The system includes, in combination, drug reservoir means containing a first drug, with the drug reservoir means in contact with the skin of the patient, and being for storing the drug and being capable of passing an electrical current through the drug wherein the drug is passed into the skin of the patient by electrokinetic action at a first skin contact area. A pair of spaced first and second electrodes is positioned in the drug reservoir means; and electrode means in electrical contact with the skin of the patient at a second skin contact area spaced from the first skin contact area, and the electrode means passes and receives current to and from the second skin contact. A first electrical circuit extends between the first and second electrodes, between the second electrode and the first skin contact area, through the skin of the patient between the first and second skin contact areas, between the second skin contact area and the electrode means, and between the said electrode means and the first electrode. The second electrical circuit includes circuit path means the second electrical circuit extending between the first and second electrodes, the second electrode and through the circuit means, and to the first electrode, the first electrical circuit, including a bypass circuit path extending between a junction positioned on the first electrical circuit the junction being positioned between the electrode means and the first electrode. First power source is positioned in the first electrical circuit between the electrode means and the junction, the first power source is for generating a first electrical current through the first circuit. A second power source is positioned in the second electrical circuit on the electrical path means for passing a second electrical current through the second circuit; and means for passing a selected current between the first and second electrodes in accordance with the dosage and type of drug in the reservoir means. Such means for passing a selected current including a plurality of selected options:

a) selectively passing only the first current in the first circuit;

b) selectively passing only the second current in the second circuit;

c) selectively inactivating the bypass circuit.

d) selectively using only the bypass circuit.

e) selecting the polarity of the first power source and

f) selecting the polarity of the second power source;

whereby a predetermined rate of electrokinetic delivery of the drug is attained.

Also included is a control system adapted to selectively operate the means for passing a selected current; and a switch positioned at the junction on the first circuit is adapted to selectively activate or deactivate the bypass circuit and simultaneously to deactivate or activate, respectively, the first circuit between the junction and the first electrode. The control system includes a computer system positioned proximate the first and second circuits, the computer system including signal circuits connected to the means for passing a selected current, computer originated output controls aligned with the signal circuits, and computer programming input circuitry capable of instructing the computer relative to operating the output controls. Also, another reservoir means includes a semi-permeable membrane capable of passing the second drug extending across the reservoir forming third and fourth reservoirs generally lateral to the skin of the patient. The third and fourth reservoirs are distal and proximate respectively to the skin of the patient, and the second drug is in generally concentrated form in the third reservoir and in generally diluted form in the fourth reservoir. Also a method of manufacturing the system described above is set forth in the detailed description that follows.

It is another object of the present invention to control the rate of delivery of a drug from a drug reservoir by controlling current flow within the drug reservoir and/or at the skin surface or mucous membrane so as to cause physical and chemical changes of the drug environment within the reservoir and/or at the skin or mucous membrane that are related to the rate of flow of the drug from the reservoir through the skin.

It is another object of the present invention to control the rate of delivery of a drug from a drug reservoir by controlling current flow within the drug reservoir and/or at the skin so as to cause physical and chemical changes of the drug within the reservoir and at the skin that are related to the rate of flow of the drug from the reservoir through the skin.

Brief Description of the Drawings

The aforementioned aspects and other features of the invention are explained in the following description taken in connection with the accompanying drawings wherein:

Figure 1 is a schematic diagram illustrating the electrode configurations applicable for controlling the rate of delivery of drug medicaments transdermally;

Figure 2 is an electrical diagram equivalent to the schematic shown in Figure 1; and

Figure 3 is a schematic diagram illustrating the movement of the drugs from the drug reservoirs and schematically illustrating a computer control mechanism in phantom lines.

Description of the Preferred Embodiments

Reference is now made to the drawings in which the same or similar elements are referred to throughout the disclosure by the same numerals.

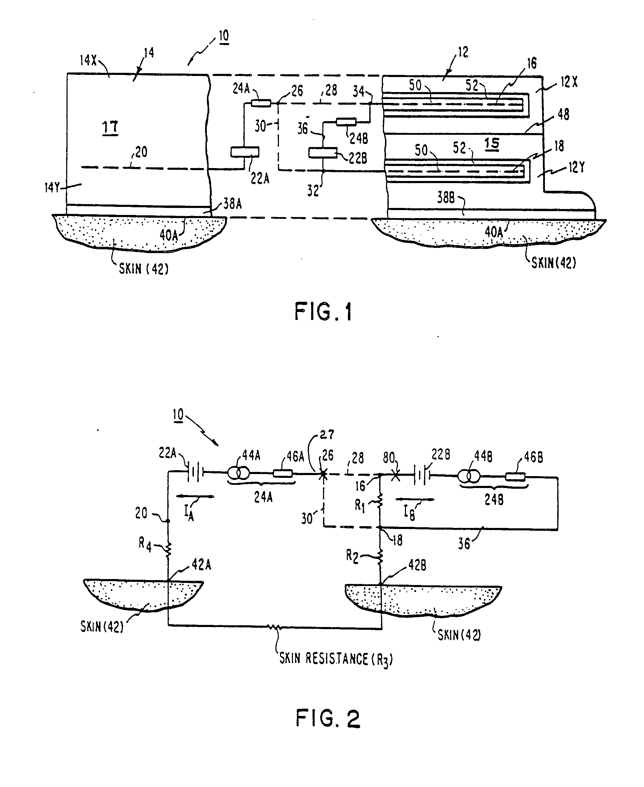

A drug applicator system 10 shown in Figures 1 and 2 includes a pair of drug reservoirs 12 and 14.

Each drug reservoir 12 and 14 preferably contains a different drug preferably contained in a gel, such as a water-soluble gel, or a suitable matrix. The gel drug mixtures are indicated as 15 and 17 in reservoirs 12 and 14 respectively. Each drug reservoir 12 and 14 includes top and bottom walls impermeable to the passage of drugs 15 and 17 and a bottom wall permeable to the passage of drugs 15 and 17.

A pair of spaced generally parallel electrodes shown here as upper electrode 16 and lower electrode 18 are positioned in reservoir 12 and a single (third) electrode 20 is positioned in reservoir 14. A first power source, such as a battery 22A, is positioned in series with electrode 20 and electrodes 16 or 18. A first current conditioning means 24A is positioned between battery 22A and electrodes 16 and 18.

A first circuit, represented by and through which a current $I_A$ flows is shown to electrically connect the skin in series with the two electrodes 16 and 18 and the third electrode 20. A by-pass electrical circuit path 30, connects the first circuit directly with lower electrode 18 by-passing upper electrode 16. The by-pass circuit extends from a junction 26 in the first circuit. An electrical path 28 extends from junction 26 to upper electrode 16 to complete the first circuit. A switch 27 is placed at junction 26 for the purpose of electrically controlling the paths 28 or 30, which as can be seen hereinafter provide the additive or subtractive nature of the current control.

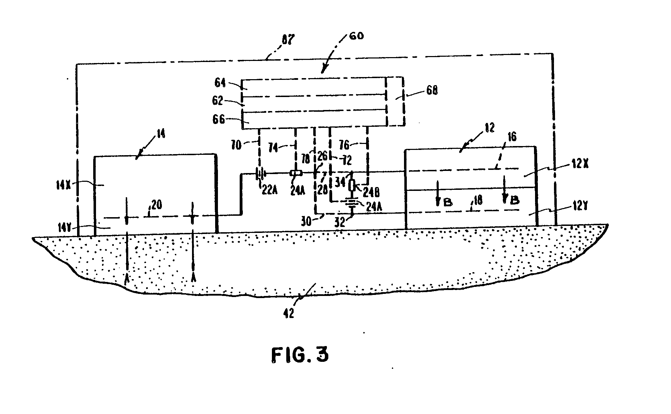

A second electrical path 36 is shown in parallel with electrodes 16 and 18. An electrical current $I_B$ passes through electrical path 36. The second electrical path is shown in Figure 1 as extending between upper junction 34 and lower junction 32 or by-pass electrical paths 28 and 30, respectively. An electrical path 36 connects junctions 32 and 34. A second power source, such as second battery 22B is in series with a second current conditioning means 24B on path 36 with the conditioning means 24B being positioned on electrical path 36. Electrically conductive membranes 38A and 38B are each capable of passing a particular drug from the bottom walls of reservoirs 14 and 12 respectively. Electrically conductive skin adhesives 40A and 40B extend along the bottom surfaces of membranes 38A and 38B respectively; adhesives 40A and 40B are in adhesive contact with the skin 42A of the patient, shown as skin or mucous membrane 42 at the electrical contact at the reservoir 14 and as skin or mucous membrane 42B at the electrical contact at the reservoir 12, respectively. Figure 2 illustrates resistances in the system as follows: $R_1$ between electrodes 16 and 18, shown in Figure 2 schematically as junctions 16 and 18; $R_2$ between electrode 18 and skin contact area 42B, $R_3$ for the skin resistance between skin contact areas 42A and 42B; and $R_4$ between skin contact area and electrode 20. Current conditioning means 24A is set forth in Figure 2 as a constant current diode 44A and timer 46A in series; and the current conditioning means 24B is set forth also as a constant current diode 44B and timer 46B in series. First battery 22A generates current $I_A$ and second battery 22B generates current $I_B$. As indicated in Figure 3, currents $I_A$ and $I_B$ are reversible in accordance with the capability of first and second batteries 22A and 22B to reverse their polarities upon command independently of one another. In addition, electrical paths 28 and 30 are either/or paths that are selectable upon predetermined command, preferably by activation of a switch 27 at junction 26 or are connected or not connected upon the circuit board or its equivalent during the manufacturing process in accordance with the drug being used and the current desired with the selected drug for prescribing a desired dosage within a particular time frame.

When batteries 22A and 22B are operating in a mode of operation wherein a current passes from reservoir 12 through resistance $R_2$ to skin contact area 42B, the current will be carried through skin 42, or mucous membrane schematically shown as skin resistance $R_3$, to skin or mucous membrane contact area 42A, through $R_3$ to electrode 20 and then to first battery 22A. When the polarity of battery 22A is reversed wherein the electrode 20 side is positive, the current described above will be completely reversed.

The above circuit provides a number of selected current options for the patient or the prescriber of the medication. These options will be set forth below. It is to be particularly noted that because of the spaced electrodes 16 and 18 in reservoir 12 and the circuits and polarities that can be selected relative to electrodes 16 and 18, a particular drug contained in reservoir 12 can be conditioned in accordance with the current options. The current options are as follows:

(a) $I_A = 0$. In this option, no current passes through skin contact area 42B at reservoir 12. $I_B$ moves in a continuous circuit in one direction or another between electrodes 16 and 18 and path 36. In this option, the current $I_B$ is used only for the purpose of using the current to change the chemistry of the drug in reservoir 12. The quantity of current $I_B$ is determined by current conditioning means 24B.

(b) Circuit 30 is selected and circuit 28 is left open by operation of a switch 27 at junction 26. Option currents $I_A$ and $I_B$ each follow independent paths, with $I_A$ travelling to skin or mucous membrane contact point 42B and $I_B$ moving in its own circuit on path 36 between electrodes 16 and 18 so as to treat the drug in reservoir 12 which $I_A$ acts to move the drug into skin or mucous membrane 42. It is to be noted that $I_A$ and $I_B$ may each be reversed by reversing the polarity of first and second batteries 22A and 22B independently of one another. There are four different sub-options of current configurations in this option.

(c) Circuit 28 is selected and circuit 30 is left open. This option results in $\pm I_A \pm I_B$ depending on which polarity is selected for batteries 22A and 22B.

(d) Circuit 28 is selected, circuit 30 is left open and $I_B = 0$. This is the most elementary of the options since $I_A$ will be used simply to move the charged drug by electrokinetic action into skin 42 at skin contact area 42A.

Electrode 20 can be positioned as shown with or without reservoir 14 but in direct electrical contact with skin contact area 42A. If reservoir 14 is used, a second drug may be placed in the reservoir different from the drug in reservoir 12. The drug in reservoir 14 would not be treated in the same manner as the drug in reservoir 12 and would migrate into skin or mucous membrane 42 at skin or mucous membrane contact area 42A by electroosmosis or by action of the ion repulsion relative to the polarity of battery 22A.

Reservoir 12 is optionally provided with a membrane 48 that separates reservoir 12 into a high concentration upper reservoir 12X and a low concentration lower reservoir 12Y. This type of reservoir separation is described in the prior U.S. patent 4 640 689.

Electrodes 16, 18 and 20 may alternately be an electrically conductive and permeable nonmetallic membrane made of a depolarizing agent. Alternately, electrodes 16 and 18 may be metallic for conductive purposes but are coated with one of the depolarizing agents such as agent 50 known in the art such as manganese dioxide ($MnO_2$) that scrubs gas formations from the electrodes so as to cause any gas formed thereon to be chemically neutralized or absorbed in the gel. In addition, an outer layer of a semi-permeable membrane 52 may be added over agent 50 so as to keep the drug in reservoir 12B away from de-polarizing agent 50.

Figure 3 illustrates in schematic form the configuration described in Figures 1 and 2. Figure 3 shows the migration of a drug A from reservoir 14 in particular from high concentration area 14X to low concentration area 14Y and from 14Y to and into skin or mucous membrane 42 of the patient. Figure 3 also shows the movement of a drug B in the reservoir 12 from high concentration area 12X to low concentration area 12Y and then from area 12Y into skin or mucous membrane 42 of the patient. A control system 60 is shown in phantom lines positioned over electric components of system 10. Control system 60 includes a computer 62 positioned between a computer battery and computer output controls 66. An optional computer input, or programming input, 68 is positioned adjacent computer 62. Signal circuits are shown between computer output controls 66 and first and second batteries 22A and 24B shown as signal circuits 70, 72, 74 and 76 respectively. A signal circuit 78 extends between junction 26, which is adapted to be a switch, so that either path 28 and/or 30 is selected at the option of the user.

Signal circuits 70 and 72 are capable of reversing the polarity of batteries 24A and 24B respectively. Signal circuit 76 is capable of controlling the current passed between electrodes 16 and 18. A switch 80 is placed in electrical path 36. This switch 80 is normally in the off position when the system is not in use, for example, during shelf-life. When the system is activated by electrical contact between the electrode 18 and skin 42, switch 80 automatically closes. The entire system with control system 60 can be located in a housing 87 shown in phantom lines.

The invention described herein can also be assembled at the place of manufacture so as to construct the plurality of optional arrangements described above in individual units in accordance with the current requirements of the drug or drugs to be placed in the drug reservoirs for administration to the patient. The method of manufacture comprises the following steps:

a) providing a first electrical circuit path through the skin having at least one reservoir containing two electrodes and a third electrode spaced from said reservoir;

b) providing a second electrical circuit path in said reservoir interconnected to said first and second electrodes;

c) providing at least one electrical power source positioned in either of said first or second electrical circuits; and

d) controlling the rate of delivery of said drug by establishing a selected current in accordance with any of the following options:

1. establishing only a first current in said first circuit path;

2. establishing only a second current in said second circuit path;

3. establishing an electrical path in said first circuit with said first electrode;

4. establishing a bypass electrical path in said first circuit with said second electrode bypassing said first electrode;

5. establishing the polarity of the said at least one power source; thus providing a predetermined rate of electrokinetic delivery for the drug.

The steps described above also include establishing a second power source in the other of the first or second electrical circuit paths. An additional step is establishing another drug reservoir about the third electrode.

The method of manufacture of the invention described herein may alternatively be described as comprising the following steps upon selection of a predetermined drug:

(a) providing a reservoir for containing the drug and placing spaced first and second electrodes in the reservoir, one side of the reservoir being provided with a permeable membrane, the first and second electrodes being spaced proximate to and distal respectively from the membrane, placing the particular drug in the reservoir and sealing the walls of the reservoir;

(b) providing a third electrode spaced from the reservoir;

(c) placing a path portion of a first electrical circuit extending from the third electrode with a junction spaced from the first electrode with a first power means and a first current conditioning means selected in accordance with the current desired in the reservoir as required by the drug, the polarity of the first power means being oriented with the current requirements;

(d) placing a second electrical circuit including a parallel circuit path with the first and second electrodes with a second power means and a second current conditioning means selected in accordance with the current desired in the reservoir as required by the drug, the polarity of the second means being oriented with the current requirements; and

(e) placing either a first electrical path between the junction and the first electrode or a second electrical path between the junction and the second electrode in accordance with the current requirement of the drug.

## Claims

1. A system for the delivery of a medicament or drug through the skin (42) or mucous membrane of a human or animal by electrokinetic action, comprising, in combination: drug reservoir means (12, 14) containing a drug, said drug reservoir means (12, 14) adapted to be in contact with said skin (42), said drug reservoir means (12, 14) retaining said drug and being capable of passing an electrical current through said drug wherein said drug is passed through the skin (42) by electrokinetic action at a first skin contact area, a pair of spaced first and second electrodes (16, 18) and circuit means for selecting the polarity of a first power source (22A) with respect to the electrodes (16, 18), characterized in that the system is intended for selectively controlling the rate of delivery of the medicament or drug, and comprises the first and second electrodes (16, 18) positioned in said drug reservoir means, said first and second electrodes being spaced apart from said first skin contact area (42B), electrode means (20) in electrical contact with the skin (42) at a second skin contact area (42A) spaced from said first skin contact area (42B), said electrode means (20) being for passing and receiving current to and from said second skin contact area (42A),

a first electrical circuit extending between said first (16) and second electrodes (18), between said second electrode (18) and said first skin contact area (42B), through the skin (42) between said first and second skin contact areas (42B, 42A), between said second skin contact area (42A) and said electrode means (20), and between said electrode means (20) and said first electrode (16),

a second electrical circuit (36) including a circuit path means, said second electrical circuit (36) extending between said first (16) and second electrodes (18), said second electrode (18) and through said circuit path means, and to said first electrode (16), said first electrical circuit, including a bypass circuit path (30) extending between a junction (26) positioned on said first electrical circuit, said junction (26) being positioned between said electrode means (20) and said first electrode (16),

the first power source (22A) positioned in said first electrical circuit between said electrode means (20) and said junction (26), said first power source (22A) being for generating a first electrical current through said first circuit,

a second power source (22B) positioned in said second electrical circuit (36) on said electrical path means adapted for passing a second electrical current through said second circuit only when current is being passed through the skin (42), and

means for passing a selected current between said first (16) and second electrodes (18) in accordance with the dosage and type of drug in said reservoir means (14, 12), said means for passing a selected current including the following options:

a) selectively passing only said first current ($I_A$) in said first circuit;

b) selectively passing only said second current ($I_B$) in said second circuit (36);

c) selectively inactivating said bypass circuit (30);

d) selectively using only said bypass circuit (30);

e) selecting the polarity of said first power source (22A); and

f) selecting the polarity of said second power source (22B);

whereby a predetermined rate of electrokinetic delivery of said drug is attained.

2. A system in accordance with claim 1, further including a control system (60) adapted to selectively operate said means for passing a selected current.

3. A system in accordance with claim 2, wherein said control system (60) includes switch means positioned at said junction (26) on said first circuit adapted to selectively activate or deactivate said bypass circuit (30) and simultaneously to deactivate or activate, respectively, said first circuit between said junction (26) and said first electrode (16).

4. A system according to claim 3, wherein said control system (60) includes a computer system (62) positioned proximate said first and second circuits, said computer system (62) including signal circuits (70, 72, 74, 76) connected to said means for passing a selected current, computer originated output controls aligned with said signal circuits, and computer programming input circuitry capable of instructing said computer relative to operating said output controls.

5. A system according to claim 3, wherein said reservoir means (12, 14) includes a semi-permeable membrane (48) capable of passing said first drug extending across said reservoir means generally between said first (16) and second electrodes (18) and forming first (12X) and second (12Y) reservoirs, said first (12X) and second (12Y) reservoirs being distal and proximate respectively to the skin (42) of the patient, and said first drug being in generally concentrated form in said first reservoir (12X) and being in generally diluted form in said second reservoir (12Y).

6. A system in accordance with claim 5, said electrode means (20) being another reservoir means (14) containing a second drug, said another reservoir means (14) being in contact with the skin (42) of the patient at said second skin contact area (42A), said electrode means (20) including a third electrode (20) positioned in said another reservoir means (14); said third electrode (20) being in electrical contact with said first power source (22A), said another reservoir means (14) being capable of passing electrical current between said third electrode (20) and said second skin contact area (42A).

7. A system according to claim 6, wherein said another reservoir means (14) includes a semi-permeable membrane capable of passing said second drug extending across said reservoir forming third and fourth reservoirs, said third and fourth reservoirs being distal and proximate respectively to the skin (42) of the patient, and said second drug being in generally concentrated form in said third reservoir and being in generally diluted form in said fourth reservoir.

8. A system according to claim 4, further including switch means positioned on said circuit path for deactivating said second electrical circuit, said switch means including switch means signal circuit connected to said output controls (66) of said computer (62), said computer programming input circuitry (68) being capable of instructing said computer (62) relative to said switch means signal circuit.

9. A system according to claim 6, wherein said first, second and third electrodes (16, 18, 20) are made of an electrically conductive and permeable non-metallic membrane containing a depolarizing agent.

10. A system according to claim 6, wherein said first, second and third electrodes (16, 18, 20) are made of metal.

11. A system according to claim 10, further including coatings for said first, second and third electrodes, said coating being a depolarizing agent (50).

12. A system according to claim 11, wherein said depolarizing agent (50) is manganese dioxide ($MnO_2$).

13. A system according to claim 14, further including outer layers over said coatings for said first, second and third electrodes (16, 18, 20), said outer layers being made of a semi-permeable membrane (52) material capable of keeping said first and second drugs from contact with said depolarizing agent.

14. A system according to claim 1, wherein said first and second current conditioning means (24A, 24B) include a constant current device (44A) and a first timer (46A) in series and a second constant current device (44B) and a second timer (46B) in series.

15. A method of delivery of a predetermined drug through the skin (42) by means of a transdermal applicator, which comprises drug reservoir means (12, 14) containing a drug, said drug reservoir means (12, 14) adapted to be in contact with said skin (42), said drug reservoir means (12, 14) retaining said drug and being capable of passing an electrical current through said drug wherein said drug is passed through the skin (42) by electrokinetic action at a first skin contact area (42B) and with a pair of spaced first and second electrodes (16, 18) characterized by the following steps:

a) providing a first electrical circuit path through the skin (42) having at least one reservoir (12) containing two electrodes (16, 18) and a third electrode (20) spaced from said reservoir (12);

b) providing a second electrical circuit path in said reservoir (12) interconnected to said first (16) and second electrodes (18);

c) providing an electrical power source (22A, 22B) in that least said first or second electrical circuits; and

d) controlling the rate of delivery of said drug by establishing a selected current in accordance with any one of the following options:

1. establishing only a first current in said first circuit path;

2. establishing only a second current in said second circuit path;

3. establishing an electrical path in said first circuit with said first electrode (16);

4. establishing a bypass electrical path (30) in said first circuit with said second current bypassing said first electrode (16);

5. establishing the polarity of the said at least one power source (22A); whereby a predetermined rate of electrokinetic delivery of said drug is attained.

16. The method according to claim 15, including a second power source (22B) in the other of said first or second electrical circuit paths.

17. The method according to claim 16, further including another drug reservoir (14) positioned about said third electrode (20).

**Patentansprüche**

1. System zum Zuführen eines Medikamentes oder einer Droge durch die Haut (42) oder Schleimhaut eines Menschen oder Tieres auf Grund einer elektrokinetischen Wirkung, welches in Kombination aufweist:

eine Droge enthaltende Drogenreservoirmittel (12, 14), welche mit der Haut (42) in Kontakt stehen können, wobei diese Drogenreservoirmittel (12, 14) die Droge zurückhalten und einen elektrischen Strom durch die Droge fließen lassen können und die Droge durch elektrokinetische Wirkung in einem ersten Hautkontaktbereich durch die Haut (42) geführt wird,

ein Paar in Abstand befindlicher erster und zweiter Elektroden (16, 18) sowie Schaltmittel zur Auswahl der Polarität einer ersten Energiequelle (22A) bezüglich der Elektroden (16, 18), dadurch gekennzeichnet, daß das System zur selektiven Steuerung der Zuführrate des Medikamentes oder der Droge bestimmt ist und die erste und zweite Elektrode (16, 18) in den Drogenreservoirmitteln gelegen sind und die erste und zweite Elektrode in Abstand von dem ersten Hautkontaktbereich (42) liegen,

Elektrodenmittel (20), die in einem von dem ersten Hautkontaktbereich (42B) in Abstand gelegenen zweiten Hautkontaktbereich (42A) in elektrischem Kontakt mit der Haut (42) stehen, wobei diese Elektrodenmittel (20) elektrischen Strom zu dem zweiten Hautkontaktbereich (42A) führen bzw. von diesem empfangen sollen,

einen ersten elektrischen Stromkreis, der zwischen der ersten (16) bzw. zweiten Elektrode (18), zwischen der zweiten Elektrode (18) und dem ersten Hautkontaktbereich (42B), durch die Haut (42) zwischen dem ersten und dem zweiten Hautkontaktbereich (42B, 42A), zwischen dem zweiten Hautkontaktbereich (42A) und dem Elektrodenmittel (20) und zwischen dem Elektrodenmittel (20) und der ersten Elektrode (16) verläuft,

einen zweiten elektrischen Stromkreis (36), der einen Strompfad beinhaltet, wobei der zweite elektrische Stromkreis (36) zwischen der ersten (16) und der zweiten Elektrode (18) und durch den Strompfad zu der ersten Elektrode (16) verläuft und der erste elektrische Stromkreis einen Bypasszweig (30) aufweist, welcher sich zwischen einer auf dem ersten elektrischen Stromkreis und zwischen dem Elektrodenmittel (20) und der ersten Elektrode (16) gelegenen Verbindungspunkt (26) und der ersten Elektrode (16) erstreckt,

daß die erste Energiequelle (22A) in dem ersten elektrischen Stromkreis zwischen dem Elektrodenmittel (20) und dem Verbindungspunkt (26) angeordnet ist und einen ersten elektrischen Strom durch den ersten elektrischen Stromkreis erzeugen soll,

eine zweite Energiequelle (22B) in dem zweiten elektrischen Stromkreis (36) auf dem Strompfad, die dazu eingerichtet ist, einen zweiten elektrischen Strom durch den zweiten Stromkreis nur dann fließen zu lassen, wenn Strom durch die Haut (42) fließt, und

Mittel, um in Obereinstimmung mit der Dosierung und der Art der Droge in den Reservoirmitteln (14, 12) einen ausgewählten Strom zwischen der ersten (16) und der zweiten Elektrode (18) fließen zu lassen, wobei die Mittel zum Fließenlassen eines ausgewählten Stromes die folgenden Alternativen aufweisen:

a) das selektive Fließenlassen nur des ersten Stromes ($I_A$) in dem ersten Stromkreis,

b) das selektive Fließenlassen nur des zweiten Stromes ($I_B$) in dem zweiten Stromkreis,

c) das selektive Inaktivieren des Bypasszweiges (30),

d) die selektive Verwendung lediglich des Bypasszweiges (30),

e) das Auswählen der Polarität der ersten Energiequelle (22A) und

f) das Auswählen der Polarität der zweiten Energiequelle (22B),

wobei eine vorbestimmte elektrokinetische Zuführrate der Droge erhalten wird.

2. System nach Anspruch 1, welches weiters ein Steuerungssystem (60) aufweist, welches zum selektiven Betreiben der Mittel zur Erzeugung eines ausgewählten Stromes dient.

3. System nach Anspruch 2, bei welchem das Steuerungssystem (60) an der auf dem ersten Stromkreis gelegenen Verbindungsstelle (26) Schaltmittel aufweist, welche zum wahlweisen Aktivieren oder Deaktivieren des Bypasszweiges (30) und gleichzeitig zum Deaktivieren bzw. Aktivieren des ersten Stromkreises zwischen der Verbindungsstelle (26) und der ersten Elektrode (16) dienen.

4. System nach Anspruch 3, bei welchem das Steuerungssystem (60) ein dem ersten und zweiten Stromkreis nahegelegenes Computersystem (62) aufweist, und das Computersystem (62) mit den Mitteln zur Erzeugung eines ausgewählten Stromes verbundene Signalschaltungen (70, 72, 74, 76) beinhaltet, weiters computererzeugte, an die Signalschaltungen angeschlossene Steuerungsausgänge sowie eine computerprogrammierende Eingangsschaltung, welche den Computer bezüglich des Betreibens der Steuerungsausgänge instruieren können.

5. System nach Anspruch 3, bei welchem das Reservoirmittel (12, 14) eine semi-permeable Membran (48) besitzt, welche für die erste Droge durchlässig ist und sich unter Bildung eines ersten (12X) und zweiten (12Y) Reservoirs im wesentlichen zwischen der ersten (16) und zweiten Elektrode (18) über die Reservoirmittel erstreckt, wobei das erste (12X) bzw. das zweite (12Y) Reservoir bezüglich der Haut (42) des Patientes distal bzw. proximal gelegen ist und die erste Droge in dem ersten Reservoir (12X) im wesentlichen in konzentrierter Form und in dem zweiten Reservoir (12Y) im wesentlichen in verdünnter Form vorliegt.

6. System nach Anspruch 5, bei welchem das Elektrodenmittel (20) ein anderes, eine zweite Droge enthaltendes Reservoirmittel (14) ist, das andere Reservoirmittel (14) in einem zweiten Hautkontaktbereich (42A) in Kontakt mit der Haut (42) des Patienten steht, das Elektrodenmittel (20) eine dritte, in dem anderen Reservoirmittel (14) angeordnete Elektrode (20) aufweist, die dritte Elektrode (20) mit der ersten Energiequelle (22A) in elektrischem Kontakt steht und das andere Reservoirmittel (14) einen elektrischen Strom zwischen der dritten Elektrode (20) und dem zweiten Hautkontaktbereich (42A) fließen lassen kann.

7. System nach Anspruch 6, bei welchem das andere Reservoirmittel (14) eine semi-permeable Membran aufweist, welche für die zweite Droge durchlässig ist und sich unter Bildung eines dritten und vierten Reservoirs über das Reservoir erstreckt, wobei das dritte bzw. vierte Reservoir bezüglich der Haut (42) des Patienten distal bzw. proximal gelegen sind und die zweite Droge in dem dritten Reservoir im wesentlichen in konzentrierter Form und in dem vierten Reservoir im wesentlichen in verdünnter Form enthalten ist.

8. System nach Anspruch 4, welches ferner auf dem Strompfad gelegene Schaltmittel zum Deaktivieren des zweiten elektrischen Stromkreises beinhaltet, wobei die Schaltmittel Schaltmittel aufweisen, die mit dem Steuerungsausgang (66) des Computers (62) über Signalschaltkreise verbunden sind und die computerprogrammierende Eingangsschaltung (68) bezüglich des Signalschaltkreises instruieren kann.

9. System nach Anspruch 6, bei welchem die erste, zweite und dritte Elektrode (16, 18, 20) aus einer elektrisch leitfähigen, durchlässigen, nicht metallischen und ein Depolarisierungsmittel enthaltenden Membran bestehen.

10. System nach Anspruch 6, bei welchem die erste, zweite und dritte Elektrode (16, 18, 20) aus Metall bestehen.

11. System nach Anspruch 10, welches weiters Beschichtungen für die erste, zweite und dritte Elektrode aufweist, wobei die Beschichtung ein Depolarisierungsmittel (50) ist.

12. System nach Anspruch 11, bei welchem das Depolarisierungsmittel Mangandioxid ($MnO_2$) ist.

13. System nach Anspruch 12, welches weiters äußere Schichten über den Beschichtungen für die erste, zweite und dritte Elektrode (16, 18, 20) aufweist, wobei die äußeren Beschichtungen aus einem semi-permeablen Material (52) bestehen, welche die erste und zweite Droge außer Kontakt mit dem Depolarisierungsmittel halten können.

14. System nach Anspruch 1, bei welchem erste und zweite stromfestlegende Mittel (24A, 24B) eine erste Konstantstromquelle (44A) und einen ersten Timer (46A) in Serie sowie eine zweite Konstantstromquelle (44B) und einen zweiten Timer (46B) in Serie enthalten.

15. Verfahren zum Zuführen einer vorbestimmten Droge durch die Haut (42) mittels eines transdermalen Applikators, der eine Droge enthaltende Drogenreservoirmittel (12, 14) aufweist, die Drogenreservoirmittel (12, 14) für einen Kontakt mit der Haut eingerichtet sind, wobei die Drogenreservoirmittel (12, 14) die Droge zurückhalten und einen elektrischen Strom durch die Droge fließen lassen können und die Droge durch elektrokinetische Wirkung in einem ersten Hautkontaktbereich (42B) durch die Haut (42) geführt wird, und mit einem Paar in Abstand befindlicher erster und zweiter Elektroden (16, 18), gekennzeichnet durch die folgenden Schritte:

a) Vorsehen eines ersten elektrischen Stromkreispfades durch die Haut (42) mit zumindest einem zwei Elektroden (16, 18) enthaltenden Reservoir (12) und einer von dem Reservoir (12) in Abstand gelegenen dritten Elektrode (20),

b) Vorsehen eines zweiten, mit der ersten (16) und zweiten Elektrode (18) verbundenen elektrischen Stromkreispfades in dem Reservoir (12),

c) Vorsehen einer elektrischen Energiequelle (22A, 22B) zumindest in dem ersten oder zweiten elektrischen Stromkreis,

d) Steuerung der Abgaberate der Droge durch Erzeugen eines ausgewählten Stromes entsprechend einer der folgenden Alternativen:

1. Erzeugen lediglich eines ersten Stromes in dem ersten Stromkreispfad,

2. Erzeugen lediglich eines zweiten Stromes in dem zweiten Stromkreispfad,

3. Herstellen eines elektrischen Pfades in dem ersten Stromkreis mit der ersten Elektrode (16),

4. Herstellen eines elektrischen Bypasspfades (30) in dem ersten Stromkreis, wodurch der zweite Strom die erste Elektrode (16) überbrückend fließt,

5. Festsetzen der Polarität der zumindest einen Energiequelle (22A), wodurch eine vorbestimmte elektrokinetische Zuführrate der Droge erhalten wird.

16. Verfahren nach Anspruch 15, welches eine zweite Energiequelle (22B) in dem anderen des ersten oder zweiten Stromkreispfades aufweist.

17. Verfahren nach Anspruch 16, welches weiters ein anderes, bei der dritten Elektrode (20) gelegenes Drogenreservoir (14) aufweist.

**Revendications**

1. Système pour la délivrance d'un médicament ou d'une drogue à travers la peau (42) ou une membrane muqueuse d'un être humain ou d'un animal par action électro-cinétique, comprenant en combinaison:

un réservoir de drogue (12, 14) contenant une drogue, ce réservoir (12, 14) étant adapté pour être en contact avec la peau (42), ce réservoir (12, 14) retenant la drogue et étant capable de faire passer un courant électrique à travers la drogue pour faire passer la drogue à travers la peau (14) par action électrocinétique sur une première zone de contact avec la peau,

une paire de première et deuxième électrodes (16, 18) espacées et un circuit pour sélectionner la polarité d'une première source de puissance (22A) par rapport aux électrodes (16, 18), caractérisé en ce que le système est prévu pour contrôler sélectivement le taux de délivrance du médicament ou de la drogue et comprend les première et deuxième électrodes (16, 18) placées dans le réservoir de drogue, les première et deuxième électrodes étant espacées par rapport à la première zone de contact avec la peau (42B), des moyens d'électrode (20) en contact électrique avec la peau (42) dans une seconde zone de contact (42A) avec la peau espacée de la première zone de contact (42B) avec la peau, les moyens d'électrode (20) servant à faire passer et à recevoir le courant vers et depuis la deuxième zone de contact avec la peau (42A),

un premier circuit électrique s'étendant entre les première (16) et deuxième électrodes (18), entre le seconde électrode (18) et la première zone de contact avec la peau (42B), à travers la peau (42) entre les première et deuxième zone de contact avec la peau (42B, 42A) entre la deuxième zone de contact avec la peau (42A) et les moyens l'électrode (20) et entre les moyens d'électrode (20) et la première électrode (16),

un deuxième circuit électrique (36) comprenant des moyens de connection de circuit, le second circuit électrique (36) s'étendant entre les première (16) et seconde électrodes (18) la seconde électrode (18), et à travers les moyens de connection des circuits, et vers la première électrode (16), le premier circuit électrique comprenant une liaison en court-circuit (30) s'étendant entre une jonction (26) placée sur le premier circuit électrique, la jonction (26) étant placée entre les moyens d'électrodes (20) et la première électrode (16),

la première source de puissance (22A) étant placée dans le premier circuit électrique entre les moyens d'électrode (20) et la jonction (26), cette première source de puissance (22A) servant à générer un premier courant électrique à travers le premier circuit,

une deuxième source de puissance (22B) placée dans le deuxième circuit électrique (36) sur les moyens de connection de circuit, destinée à faire passer un second courant électrique à travers le second circuit seulement lorsque le courant passe à travers la peau, et

des moyens pour faire passer un courant sélectionné entre les première (16) et seconde électrodes (18) selon le dosage et le type de drogue dans le réservoir (14, 12), des moyens pour faire passer le courant sélectionné comprenant les options suivantes:

a) faire passer sélectivement seulement le premier courant ($I_A$) dans le premier circuit,

b) sélectivement faire passer le second courant ($I_B$) dans le second circuit (36),

c) sélectivement rendre inactif la liaison en cours circuit (30),

d) sélectivement utiliser seulement la liaison en court circuit (30),

e) sélectionner la polarité de la première source de puissance (22A), et

f) sélectionner la polarité de la deuxième source de puissance (22B), par lesquelles un taux prédéterminé de délivrance électrocinétique de la drogue est atteint.

2. Système selon la revendication 1, comprenant en plus un système de contrôle (60) adapté pour actionner sélectivement les moyens pour faire passer un courant sélectionné.

3. Système selon la revendication 2, par lequel un système de contrôle (60) comprend des moyens d'interrupteur placés à la jonction (26) sur le premier circuit destinés à activer ou désactiver sélectivement la liaison en court circuit (30) et simultanément à désactiver ou activer, respectivement, le premier circuit entre la jonction (26) et la première électrode (16).

4. Système selon la revendication 3, dans lequel le système de contrôle (60) comprend un système informatique (62) placé à proximité des premier et second circuits, le système informatique (62) comprenant des circuits de signaux (70, 72, 74, 76) reliés au moyen faisant passer le courant sélectionné, des contrôles de sortie en provenance de l'ordinateur alignés avec les circuits de signaux, et un circuit d'entrée pour le programme d'ordinateur capable d'instruire l'ordinateur sur la façon d'actionner les contrôles de sortie.

5. Système selon la revendication 3, dans lequel le réservoir (12, 14) comprend une membrane semi-perméable (48) capable de laisser passer la première drogue contenue dans le réservoir généralement entre les première (16) et deuxième électrode (18) et formant un premier (12X) et deuxième (12Y) réservoirs, les premier (12X) et deuxième (12Y) réservoirs étant distaux et à proximité respectivement de la peau du patient et la première drogue étant en général sous forme concentrée dans le premier réservoir (12X) et étant en général sous forme diluée dans le deuxième réservoir (12Y).

6. Système selon la revendication 5, dans lequel les moyens d'électrodes (20) constitue un autre réservoir (14) comprenant une deuxième drogue, cet autre réservoir (14) étant en contact avec la peau (42) du patient sur la deuxième zone de contact avec la peau (42A), moyens d'électrodes (20) comprenant une troisième électrode (20) placée dans l'autre réservoir (14), la troisième électrode (20) étant en contact électrique avec la première source de puissance (22A), cet autre réservoir (14) étant capable de laisser passer le courant électrique entre la troisième électrode (20) et la seconde zone de contact avec la peau (42A).

7. Système selon la revendication 6, dans lequel l'autre réservoir (14) comprend une membrane semi-perméable capable de laisser passer une deuxième drogue contenue dans le réservoir formant un troisième et quatrième réservoir, ces troisième et quatrième réservoir étant distaux et à proximité respectivement de la peau (42) du patient, la deuxième drogue étant en général sous forme concentrée dans le troisième réservoir et étant en général sous forme diluée dans le quatrième réservoir.

8. Système selon la revendication 4, comprenant en plus des moyens d'interruption placée sur le circuit de connection pour rendre inactif le le deuxième circuit électrique, ces moyens de commutation comprenant un circuit de signal de commutation relié au contrôle de sortie (66) de l'ordinateur (62), le circuit (68) d'entrée du programme de l'ordinateur étant capable d'instruire l'ordinateur (62) en ce qui concerne le circuit de signal de commutation.

9. Système selon la revendication 6, par lequel les première, seconde et troisième électrodes (16, 28, 20) sont faites dans une membrane non-métallique électriquement conductrice et perméable, contenant un agent dépolarisant.

10. Système selon la revendication 6, dans lequel, les première, deuxième et troisième électrode (16, 18, 20) sont métalliques.

11. Système selon la revendication 10, comprenant de plus un des revêtements sur les première, deuxième et troisième électrodes, le revêtement étant un agent dépolarisant (50).

12. Système selon la revendication 11, dans lequel l'agent dépolarisant (50) est du dioxyde de manganèse ($MnO_2$).

13. Système selon la revendication 12 comprenant en plus une couche extérieure par dessus les revêtements des première, seconde et troisième électrode (16, 18, 20), ces couches extérieures étant faites dans un matériau de membrane semi-perméable (52) capable d'empêcher le contact des première, deuxième drogue avec l'agent dépolarisant.

14. Système selon la revendication 1, caractérisé en ce que les premier et deuxième moyens de conditionnement du courant comprennent un dispositif à courant constant (44A) et une première minuterie (46A) en série et un deuxième dispositif à courant constant (44B) et une seconde minuterie (46B) en série.

15. Méthode de délivrance d'une drogue prédeterminée à travers la peau (42) au moyen d'un applicateur transdermique, qui comprend un réservoir de drogue (12, 14) contenant une drogue, ce réservoir (12, 14) étant adapté pour être en contact avec la peau (42), le réservoir de drogues (12, 14) retenant la drogue et étant capable de laisser passer un courant électrique à travers la drogue quand la drogue passe à travers la peau par action électrocinétique sur une première zone de contact avec la peau (42B), et avec une paire de première et seconde électrodes espacées (16, 18), caractérisé par les étapes suivantes:

a) fournir un premier circuit de connection électrique à travers la peau (42) présentant au moins un réservoir (12) contenant deux électrodes (16, 18) et une troisième électrode (20) espacé du réservoir (12);

b) fournir un second circuit de connection électrique dans le réservoir (12) interconnecté aux première (16) et seconde électrodes (18);

c) fournir une source de puissance électrique (22A, 22B) dans au moins le premier ou le second circuit électrique;

d) contrôler le taux de délivrance de la drogue en établissant un courant sélectionné selon l'une des options suivantes:

1. établir seulement un premier courant dans le premier circuit de connection;

2. établir seulement un deuxième courant dans le deuxième circuit de connection;

3. établir un circuit électrique le premier circuit avec la première électrode (16);

4. établir une liaison électrique en court circuit (30) dans le premier circuit avec le deuxième courant courtcircuitant la première électrode (16);

5. établir la polarité d'au moins une des sources de puissance (22A), par lesquelles un taux prédéterminé de délivrance électrocynétique de la drogue est atteint.

16. Méthode selon la revendication 15, comprenant une deuxième source de puissance (22B), dans l'autre du premier ou deuxième circuit de connection électrique.

17. Méthode selon la revendication 16, comprenant en outre un autre réservoir de drogues (14) placé près de la troisième électrode (20).

FIG.1

FIG. 2

FIG. 3